# FASCICULE DE BREVET EUROPEEN

(11) **EP 2 027 463 B1**
(45) Date de publication et mention de la délivrance du brevet: **21.11.2012**
(21) Numéro de dépôt: 06847119.2
(22) Date de dépôt: 22.12.2006
(51) Int. Cl.: G01N 33/00, G08B 21/14

(54) **PROCEDE DE MESURE ET D'ALERTE FIABILISEES ET INDIVIDUALISEES DE POLLUTIONS DE L'AIR ET DISPOSITIF ASSOCIE**
VERFAHREN ZUR ZUVERLÄSSIGEN, INDIVIDUALISIERTEN MESSUNG VON UND WARNUNG VOR LUFTVERSCHMUTZUNG SOWIE DAMIT ZUSAMMENHÄNGENDE VORRICHTUNG
METHOD FOR RELIABLE, INDIVIDUALIZED MEASUREMENT AND WARNING OF AIR POLLUTION, AND ASSOCIATED DEVICE

(30) Priorité: 26.12.2005 FR 0513398
(43) Date de publication de la demande: 25.02.2009
(73) Titulaire: Aubert, Bruno, 30330 Connaux (FR)
(72) Inventeur: Aubert, Bruno, 30330 Connaux (FR)
(74) Mandataire: Fourcade, Emmanuelle
(86) Numéro de dépôt international: PCT/FR2006/002848
(87) Numéro de publication internationale: WO 2007/077354

(56) Documents cités:
- EP-A- 0 280 540
- DE-C1- 4 002 843
- US-A- 2 234 499
- US-A- 4 406 770
- US-A- 4 633 704
- US-A- 5 376 554

## Description

La présente invention a pour objets un procédé de mesure et d'alerte de pollutions de l'air en vue de prévenir les personnes, notamment les asthmatiques, des risques encourus si elles s'exposent dehors à un air pollué et son dispositif associé.

Ledit procédé convient, comme cela va ressortir de son descriptif, pour détecter dans son environnement immédiat, les polluants dans l'air que l'on respire, qui seraient au delà d'un seuil jugé dangereux par les services de santé, et pour avertir les personnes individuellement.

Il est connu que les personnes présentant des gênes respiratoires, comme les asthmatiques, en présence de polluants, doivent limiter leurs sorties en cas de 10 dépassement de seuils de certains polluants et même limiter leur activité physique. Jusqu'à présent, lorsqu'une personne asthmatique souhaitait savoir si elle pouvait sortir de chez elle sans risque vis-à-vis des polluants tels que l'ozone, les oxydes d'azote ou de soufre, elle devait s'informer (télévision, radio...) sans être sûr qu'un pic de pollution ne survienne sporadiquement dans la journée.

15 De plus, lorsque la personne est loin de toute source d'information, elle ne dispose d'aucune autre solution de prévention.

S'il existe des appareils de mesure de la teneur en ozone, oxydes d'azote ou de soufre, notamment à base de spectromètres, ils sont très gros et ne peuvent en aucun cas être portés. De plus, leur complexité rendrait leur utilisation impossible par des enfants.

20 Il existe aussi de petits appareils de prélèvement en vue d'une analyse, mais le résultat est disponible bien trop tard car il faut envoyer l'échantillon à un laboratoire.

Enfin, il existe bien de petits appareils de mesure de la pollution au moyen de capteurs électroniques ou d'indicateurs colorimétriques qui changent de couleur en présence de polluants spécifiques, mais de nombreux paramètres comme l'humidité (qui absorbe 25 l'ozone), viennent perturber la mesure dans des proportions significatives (d'un facteur 1 à 10) et rend impossible une prévention fiable des personnes fragiles. Le document EP028054DA décrit un tel capteur comprenant des moyens de limiter l'interférence de certains gaz par leur combustion préalable.

La présente invention permet d'éliminer le problème principal de la méconnaissance de la présence d'un risque pour les personnes et notamment les asthmatiques, par un procédé et un dispositif miniature simple et fiable qui les avertira de façon certaine en cas de dépassement de seuil des polluants.

Le procédé objet de la présente invention consiste à faire préalablement circuler l'air dont on veut mesurer s'il contient un polluant déterminé au-delà d'un seuil jugé dangereux pour les personnes, à travers ou sur des matériaux absorbants les composés de l'air qui interfèrent dans la mesure de la pollution.

35 Ces matériaux absorbants sont mis en oeuvre sous la forme de patches ou de membranes poreuses auto-fixant grâce à des dispositifs type magnétique, mécanique, colle réversible ou tissu auto-agrippant et sont jetables après usage (par exemple de façon préventive une fois par jour, par semaine ou par mois en fonction de la quantité d'absorbant utilisé ou dès que leur porosité est diminuée de façon trop importante du fait des polluants ou des poussières ce qui peut être mis en évidence par une perte de charge trop importante au niveau de la pompe à air).

Dans le cas ou l'air traverse le patch ou la membrane, les divers matériaux absorbants les interférents peuvent être disposés en multicouche. Dans le cas où l'air ne fait que balayer le patch ou la membrane, ces matériaux absorbants seront mélangés et disposés sous la forme d'une seule couche.

A titre d'exemple, l'un des matériaux constitutif du patch ou de la membrane poreuse pourra être un gel de silice qui absorbe l'humidité ou du coton hydrophile.

La mesure de la teneur en ozone par exemple sera donc précise par temps sec comme sous la pluie.

Pour absorber les oxydes d'azote ou de soufre, des charbons spécifiquement activés vis-à-vis de ces molécules seront mis en oeuvre.

D'autre part, ces matériaux absorbants doivent être réversibles pour pouvoir libérer tout ou partie des gaz absorbés une fois qu'ils se trouvent dans un environnement plus pauvre en ce gaz ceci afin d'une part de régénérer le dit matériau absorbant et d'autre part permettre des variations douces des concentrations des gaz interférents.

Par exemple pour l'humidité, le matériau hydrophile comme le coton va absorber l'humidité excessive lorsque l'on passe de l'extérieur (où des humidités de 10% ne sont pas rares en hiver) à l'intérieur d'un bâtiment (où l'humidité peut atteindre 90%). Puis lorsque la personne retourne à l'extérieur, le coton va libérer son humidité pour que le changement d'humidité de l'air allant sur le capteur soit progressif.

Ainsi, ce véritable prétraitement de l'air permet d'assurer les mêmes conditions optimales de mesure du capteur électronique ou de l'indicateur colorimétrique quelque soit les conditions atmosphériques.

Une alarme visuelle, sonore ou vibrante, fiable pourra ensuite être donnée via un circuit électronique gérant les niveaux d'alertes.

Avantageusement un revêtement hydrophobe sera disposé entre l'air à mesurer et le patch ou membrane (sur la couche la plus extérieure) afin de permettre un fonctionnement sous la pluie ou lorsque l'on se lave les mains.

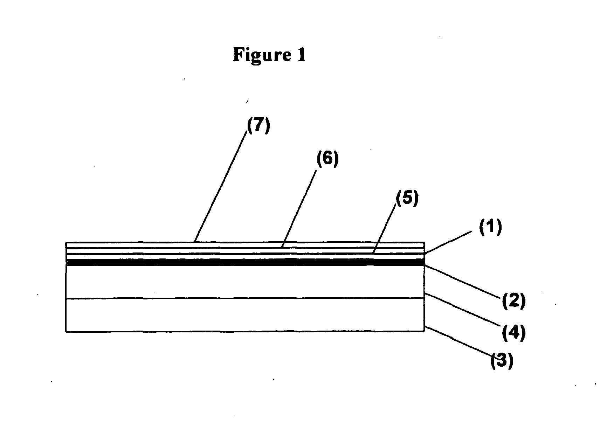

La figure 1 montre la coupe d'un exemple de dispositif selon l'invention avec un indicateur colorimétrique et une membrane microporeuse pour que l'air passe au travers.

La membrane microporeuse transparente (1) est constituée de différentes couches, elles aussi microporeuses et transparentes, de matériaux absorbants les composés de l'air qui interfèrent dans la mesure de la pollution. Cette membrane est posée directement sur un indicateur colorimétrique lui-même microporeux (2).

La première couche (7) est un matériau hydrophobe pour permettre un fonctionnement sous la pluie.

La deuxième couche (6) est un absorbant de N02 et S02 à base de charbon activé.

La troisième couche (5) est un absorbant d'humidité à base de gel de silice.

Une micro pompe piézoélectrique (4) reliée à la pile (3) permet d'assurer un débit régulier au travers de la membrane et de l'indicateur colorimétrique micro poreux.

La surveillance de l'intensité alimentant la pompe permet de savoir si le patch ou la membrane est colmaté.

Dans le cas de l'utilisation d'un capteur électronique, le patch ou la membrane microporeux peut être disposée directement sur la pompe qui envoie l'air traité vers le capteur.

Le patch ou la membrane peut également être non poreux auquel cas un espace est aménagé entre le patch et la pompe afin que l'air qui est dirigé vers le capteur circule bien sur toute la surface du patch ou de la membrane pour être débarrassé des interférents.

Le capteur est aussi relié à un circuit électronique qui va déclencher une diode électroluminescente, un affichage à cristaux liquides, un vibreur ou un buzzer en cas de dépassements de seuil.

La miniaturisation des composants comme les micro-pompes piézoélectriques permet de proposer un appareil de la taille d'une pièce de un euro.

Une première application est un dispositif d'alerte individualisée portable sur le revers d'une veste ou au poignet, qui alerte les asthmatiques de la présence d'un pic de pollution à l'ozone, aux oxydes de soufre ou d'azote.

Périodiquement, l'utilisateur met un nouveau patch ou une nouvelle membrane absorbants sur son moyen de mesure afin d'être sûr d'être bien prévenu d'une éventuelle pollution.

## Revendications

1. Procédé de mesure et d'alerte fiabilisées et individualisées de pollutions de l'air, **caractérisé en ce que** préalablement à la mesure permettant de donner l'alerte, on ôte les composés de l'air qui interfèrent dans la dite mesure au moyen de matériaux absorbants sélectifs réversibles sous forme de patches ou de membranes auto-fixant jetables après usage et disposés entre l'air à mesurer et les moyens de mesure.
- 2- Dispositif de mise en oeuvre du procédé suivant la revendication (1), **caractérisé en ce qu'**un des matériaux qui absorbent les composés de l'air qui interfèrent dans la mesure de la pollution est un matériau (5) hydrophile tels les gels de silice ou le coton et/ou qu'un autre est un matériau qui absorbe les oxydes d'azote et/ou les oxydes de soufre (6).
- 3- Dispositif selon la revendication (2), **caractérisé en ce qu'**un matériau hydrophobe est disposé entre l'air à mesurer et les patches ou membranes.
- 4- Dispositif selon l'une des revendications (2) à (3), **caractérisé en ce qu'**une pompe à air assure un débit régulier de l'air à mesurer vers les patches et les moyens de mesure.
- 5- Dispositif selon l'une des revendications (2) à (4), **caractérisé en ce que** les moyens de mesure sont reliés à un circuit électronique gérant les alertes en fonction des seuils réglementaires des polluants.
- 6- Dispositif selon l'une des revendications (2) à (5), **caractérisé en ce que** le circuit électronique permet la détection d'une augmentation de la perte de charge au niveau de la pompe à air en vue d'avertir l'utilisateur d'un nécessaire changement de patches ou de membrane.
- 7- Dispositif suivant l'une quelconque des revendications (2) à (6), **caractérisé en ce qu'**il comprend une micro-pompe, un capteur de pollution, un circuit électronique gérant les alarmes et un patch recouvert de matériaux absorbant les interférents de mesure de pollution et qu'il est mis en oeuvre pour alerter individuellement les personnes d'un dépassement d'un seuil de pollution de l'air, nuisible pour la santé.

## Claims

1. Reliable, individual process for measuring air pollution and generating alerts **characterized in that** any air components that may interfere with the measuring process are removed before the reading is taken, using disposable adhesive patches or membranes made of selective reversible absorbent materials that are inserted between the air intake and the measuring device.

2. Device for implementing the process described in claim (1) **characterized in that** one of the materials used to absorb air components that may interfere with the pollution reading is a hydrophilic material (5) like silica gel or cotton, and/or another absorbs nitrogen oxide and/or sulphur oxide (6).

3. Device as described in claim (2) **characterized in that** a hydrophobic material is placed between the air intake and the patches or membranes.

4. Device as described in one of claims (2) and (3) **characterized in that** an air pump is used to ensure regular throughput of air towards the patches and measuring device.

5. Device as described in one of claims (2) to (4) **characterized in that** the measuring device is linked to an electronic circuit that generates alerts if a reading exceeds official pollution thresholds.

6. Device as described in one of claims (2) to (5) **characterized in that** the electronic circuit detects any increased loss of load in the air coming through the air pump, and notifies the user that the patches or membranes must be changed.

7. Device as described in one of claims (2) to (6), which comprises a micro-pump, pollution sensor, electronic circuit used to generate alerts, and a patch made from materials that absorb components that may interfere with the pollution reading, and which is used to notify individual users that an air pollution threshold has been crossed with possible adverse effects for health.

## Patentansprüche

1. Verlässliches, individuelles Verfahren zur Schadstoffmessung und -warnung, wonach vor der Messung, die den Alarm auslöst, störende Luftbestandteile herausgefiltert werden. Dies geschieht mit Hilfe von absorbierenden, selektiven und austauschbaren Materialien in Form von selbstklebenden Einmal-Filtern oder Membranen, die zwischen der Messluft und den Messgeräten angebracht werden.

2. Maßnahme zum Einsatz des Verfahrens gemäß Anforderung 1, wonach eines der Materialien (5), das die bei der Schadstoffmessung störenden Luftbestandteile herausfiltert, hydrophil ist, wie z.B. Siliziumgel oder Watte und/ oder dass ein weiteres Material Stickoxide und/ oder Schwefeloxide absorbiert. (6).

3. Maßnahme gemäß Anforderung 2, wonach ein hydrophobes Material zwischen Messluft und Filtern oder Membranen eingesetzt wird.

4. Maßnahme gemäß einer der Anforderungen 2 bis 3, wonach eine Luftpumpe für eine regelmäßige Zufuhr an Messluft in Richtung Filter und Messgeräte sorgt.

5. Maßnahme gemäß einer der Anforderungen 2 bis 4, wonach die Messgeräte an einen elektronischen Schaltkreis angeschlossen sind, der den Alarm je nach vorgeschriebenen Schadstoffgrenzwerten auslöst.

6. Maßnahme gemäß einer der Anforderungen 2 bis 5, wonach mit Hilfe des elektronischen Schaltkreises ein erhöhter Druckabfall der Luftpumpe festgestellt und dem Benutzer somit ein notwendiger Filter- oder Membranwechsel angezeigt werden kann.

7. Maßnahme gemäß einer beliebigen Anforderung zwischen 2 und 6, wonach das Gerät eine Mikropumpe, einen Schadstoffsensor, einen elektronischen Schaltkreis für die Alarmfunktion sowie einen Filter mit einer Materialbeschichtung enthält, welche die bei der Schadstoffinessung störenden Luftbestandteile absorbiert, und das Gerät individuell bei Personen zur Warnung vor einer Grenzwertüberschreitung gesundheitsgefährdender Luftschadstoffe zum Einsatz kommt.
